(19) Europäisches Patentamt
European Patent Office
Office européen des brevets

(11) Veröffentlichungsnummer: **0 310 948**
**A2**

(12) # EUROPÄISCHE PATENTANMELDUNG

(21) Anmeldenummer: **88116109.5**

(51) Int. Cl.⁴: **C12P 41/00 , C12P 13/04**

(22) Anmeldetag: **29.09.88**

(30) Priorität: **03.10.87 DE 3733506**

(43) Veröffentlichungstag der Anmeldung:
**12.04.89 Patentblatt 89/15**

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Hoppe, Hans-Ulrich, Dr.**
**49 Rue Villebois-Mareuil**
**F-95460 Ezanville(FR)**
Erfinder: **Schlingmann, Merten, Prof. Dr.**
**Schneidhainer Strasse 32a**
**D-6240 Königstein/Taunus(DE)**
Erfinder: **Keller, Reinhold, Dr.**
**Kaunweg 8**
**D-6232 Bad Soden am Taunus(DE)**

(54) **Verfahren zur Herstellung optisch reiner phenylringsubstituierter Phenylalaninderivate.**

(57) Optisch reine phenylringsubstituierte Phenylalaninderivate können durch racemische Spaltung mit Hilfe von Subtilisin bei schwach saurem bis schwach alkalischem pH hergestellt werden, wobei der D-Amidoester extrahiert und zur D-Aminosäure verseift wird und die verbliebene L-Amidosäure nach Ansäuern extrahiert und in die L-Aminosäure überführt wird.

EP 0 310 948 A2

Xerox Copy Centre

## Verfahren zur Herstellung optisch reiner phenylringsubstituierter Phenylalaninderivate

Die Erfindung betrifft ein Verfahren zur Herstellung nicht proteinogener, optisch reiner phenylringsubstituierter Phenylalaninderivate, das auf der enzymatischen Racematspaltung solcher racemischen Aminosäuren beruht.

Die optisch reinen Aminosäuren können beispielsweise zur Herstellung von optisch reinen Penicillinen und Cephalosporinen dienen, die bisher nur als Epimeren-Gemische zur Verfügung standen. Weiterhin können diese optisch reinen Aminosäuren zur herstellung bisher nicht bekannter Penicilline und Cephalosporine verwendet werden oder natürliche Aminosäuren in synthetischen bzw. natürlichen Peptiden ersetzen.

Enzymatische Racematspaltungen von proteinogenen Aminosäuren sind bereits beschrieben.

Aus der deutschen Offenlegungsschrift 2 215 853 ist es bekannt, racemische, mit 3-$OCH_3$, 2-, 3-, 4- oder 3,4-OH, 4-Cl und 4-F ringsubstituierte Phenylalanine in die entsprechenden L-Aminosäuren dadurch zu überführen, daß man das Racemat zunächst mit einem Alkanol mit bis zu 4 Kohlenstoffatomen verestert, den racemischen Ester der Einwirkung eines Chymotrypsins bei einem sauren pH unterwirft, die L-Aminosäure durch Fällung isoliert und gegebenenfalls den Ester der D-Aminosäure aus dem Filtrat extrahiert und verseift. Als Enzym wird das α-Chymotrypsin bevorzugt; der bevorzugte pH-Bereich bei der enzymatischen Spaltung liegt bei 5 bis 6. Die nach Abtrennung der L-Aminosäure verbliebene Flüssigkeit wird vor der Extraktion des Esters der D-Aminosäure alkalisch gestellt.

In der deutschen Offenlegungsschrift 34 38 189 wird ein Verfahren zur Herstellung von aromatisch substituierten L-Aminosäuren durch Spaltung der racemischen niederen Alkylester mit α-Chymotrypsin im schwach sauren wäßrigen Medium beschrieben, bei dem das Reaktionsgemisch schwach alkalisch gestellt wird, der Ester der D-Aminosäure mit einem organischen Lösungsmittel extrahiert, racemisiert und wieder in den Prozeß zurückgeführt wird, und die L-Aminosäure aus dem das wäßrigen alkalischen Extrakt nach Ansäuern isoliert wird.

In der deutschen Offenlegungsschrift 29 27 534 sind optisch reine heterocyclische α-Aminosäuren beschrieben sowie ein Verfahren zur Herstellung dieser Verbindungen, das auf der Umsetzung eines racemischen α-Aminosäurederivats mit einem trägergebundenen, proteolytischen Enzym beruht. Als verwendbare Enzyme werden unter anderem Subtilisin und α-Chymotrypsin beschrieben.

Gemäß der deutschen Offenlegungsschrift 29 27 534 wird für die racemische Spaltung von Derivaten natürlicher, vornehmlich in Proteinen vorkommenden Aminosäuren, die Anwendung von proteolytischen Enzymen wie Subtilisin und Chymotrypsin, empfohlen. Es wurde angenommen, daß die Substratspezifität von Subtilisin und Chymotrypsin in Bezug auf die Derivate natürlicher Aminosäuren nahezu identisch ist.

Bei den Versuchen N-acylgeschütztes ortho-Trifluormethylphenylalanin - ein Derivat einer natürlichen Aminosäure -racemisch mit Chymotrypsin zu spalten, konnte die in der Deutschen Offenlegungsschrift 29 27 534 beschriebene vorhersehbare Spezifität von Chymotrypsin nicht bestätigt werden. Ortho-Trifluormethyl-phenylalanin läßt sich nicht mittels Chymotrypsin spalten. Weitere Versuche mit Subtilisin scheiterten ebenfalls.

Auch andere ringsubstituierte Phenylalanine (z.B. para-Trifluormethyl-phenylalanin) lassen sich mit Chymotrypsin nicht oder nur in sehr geringen Ausbeuten racemisch spalten.

Es wurde festgestellt, daß die Substratspezifität der beiden proteolytischen Enzyme Subtilisin und Chymotrypsin keineswegs - wie bisher angenommen - nahezu identisch ist.

Die Erfindung betrifft nunmehr ein Verfahren zur Herstellung optisch reiner phenylringsubstituierter Phenylalaninderivate durch racemische Spaltung mittels eines proteolytischen Enzyms, das dadurch gekennzeichnet ist, daß eine racemische Verbindung der Formel I,

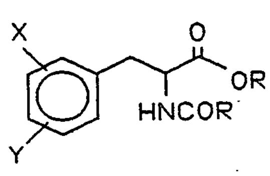

(I)

in der

X und Y gleich oder verschieden sind und Wasserstoff, Fluor, Chlor, Brom, ($C_1$-$C_4$)-Alkyl oder Trifluormethyl bedeuten und

R  $(C_1\text{-}C_4)$-Alkyl und
R′  eine $(C_1\text{-}C_4)$-Alkyl- oder eine Benzylschutzgruppe bedeutet,

wobei X und Y niemals gleichzeitig Wasserstoff bedeuten und der Trifluormethyl-Substituent niemals die ortho-Position des Phenylrings besetzt, der Einwirkung von Subtilisin (EC 3.4.4.16) bei schwach saurem bis schwach alkalischem pH unterworfen wird.

Außerdem betrifft die Erfindung die Verwendung der optisch reinen Aminosäuren zur Herstellung von Penicillinen, Cephalosporinen oder Peptiden.

Die Verbindungen der Formel I sind N- und O-geschützten phenylringsubstituierte Phenylalaninderivate, die im Phenylring (des Phenylalanin) mit Fluor, Chlor, Brom, $(C_1\text{-}C_4)$-Alkyl und/oder Trifluormethyl mono- oder disubstituiert sind und deren Carboxy- und Aminofunktion mit einer Schutzgruppe geschützt sind. Als Schutzgruppen kommen prinzipiell solche in Betracht, die auch in der Peptidchemie bei Aufbau von Peptiden Verwendung finden. Als Amino-Schutzgruppen werden bevorzugt Carbonyloxyalkyle mit 1-4 Kohlenstoffatomen, insbesondere der Acetylrest eingesetzt. Bevorzugte Carboxy-Schutzgruppen sind Alkyle mit 1-4 Kohlenstoffatomen, insbesondere die Methylgruppe oder die Benzylgruppe. Bevorzugt werden die Verbindungen der Formel I eingesetzt, in denen
X Fluor in ortho-, meta- oder para-Position oder Trifluormethyl in meta- oder para-Position oder Brom in para-Position und Y Wasserstoff bedeutet oder X Chlor in para-Position und Y Trifluormethyl in meta-Position bedeutet oder X n-Butyl in para-Position und Y Wasserstoff bedeutet oder X Fluor in para-Position und Y Fluor in ortho-Position bedeutet und die Schutzgruppen die obengenannte Bedeutung haben.

Die racemischen Gemische der Verbindungen der Formel I sind - sofern nicht käuflich - auf einfache Weise nach literaturbekannten Verfahren erhältlich. Beispielsweise können sie aus den entsprechend substituierten Benzaldehyden mit N-Acylglycinen nach Erlenmeyer über den "Azlactonweg" hergestellt werden (siehe z.B.: Org. Reactions Vol. III, S. 198 (1947) und dort zitierte Literatur; Houben-Weyl Bd 11/2, S. 458 (1958)).

Das erfindungsgemäße Verfahren beruht auf der selektiven Hydrolyse der L-Phenylalaninderivate der allgemeinen Formel I mittels Subtilisin (EC 3.4.4.16). Da die D-Verbindungen dieser Hydrolyse nicht unterliegen, können sie auf diese Weise leicht von den L-Verbindungen abgetrennt werden.

Die enzymatische Spaltung erfolgt vorzugsweise bei einer Temperatur von 30-65 °C, insbesondere bei 43-47 °C in einem pH-Bereich, der von schwach sauer bis schwach alkalisch variieren kann. Bevorzugt ist ein pH-Bereich von 5,5-8,5, insbesondere arbeitet man bei einem pH-Wert von 7,0. Das Substrat, die racemische Verbindung der Formel I, wird als 15-25 Gew.-%ige, bevorzugt 19-21 Gew.-%ige Lösung mit dem 3-5-fachen, bevorzugt 4-fachen Volumen an Wasser versetzt und anschließend mit 60-600 U, bevorzugt 100-300 U Subtilisin pro 100 g Substrat (®Maxatase Hersteller: Gistbrocades N.V. Delft/Niederlande; ®Optimase, Hersteller: Miles-Kali-Chemie, Hannover; ®Alcalase, Hersteller: Novo Industri AS, Kopenhagen/Dänemark) inkubiert und während der enzymatischen Umsetzung intensiv gerührt. (Die Aktivität des Enzyms wird nach den üblichen Methoden mit Dimethylcasein als Substrat bei 50 °C und pH 8.3 bestimmt.) Der Verlauf und der Endpunkt der Reaktion kann durch pH-Wert-Messung bzw. durch die notwendige Neutralisation der entstehenden $H^+$-Ionen bestimmt werden. Die Neutralisation bzw. die Einhaltung des pH-werts in den angegebenen Grenzen erfolgt durch Basenzusatz.

Das Substrat - die Verbindung der Formel I - kann beispielsweise in Wasser, Ethanol, Methanol oder beliebigen Wasser-Alkohol-Mischungen, bevorzugt jedoch in einem mit Wasser nicht oder nur begrenzt mischbaren Lösungsmittel wie Methylisobutylketon (MIBK) oder Essigester gelöst werden (zweiphasiges System).

Das Enzym Subtilisin kann sowohl als solches, als auch gebunden an einen Träger, eingesetzt werden (immobilisiertes Enzym). Als Enzymträger kommen beispielsweise anorganische Träger wie Aluminiumsilikate aber auch polymere, organische Träger wie Cellulosen, modifizierte Polyacrylamidgele, die Amino- oder Hydroxylgruppen besitzen oder auch organische Copolymerisate aus Acrylamid, Metacrylaten oder Metacrylamid und Maleinsäureanhydrid in Frage. Die Herstellung entsprechender trägergebundener Enzyme ist beispielsweise beschrieben in Halwachs et al., Biotechnology and Bioengineering XIX (1977) 1667-1677 (Fixierung auf Aluminiumsilikat) oder DE-OS 29 27 534 (Fixierung auf Cellulose).

Bevorzugt wird Maxatase als solche, d.h. als wäßrige Lösung eingesetzt. Maxatase stellt eine rohe und preiswerte Subtilisin-Qualität für Waschmittelzwecke dar.

Bei der enzymatischen Spaltung der N- und O-geschützten racemischen Aminosäure wird allein der Ester der L-Verbindung hydrolysiert. Es entsteht also ein Gemisch aus L-N-acyl-aminosäure (= L-Amidosäure) und D-N-acylaminosäureester (= D-Amidoester). Diese beiden optisch aktiven Verbindungen können auf Grund ihrer unterschiedlichen Löslichkeiten in wäßrigen bzw. organischen Lösungsmitteln voneinander getrennt werden.

Zur Aufarbeitung der nach der enzymatischen Spaltung erhaltenen wäßrigen Lösung kann beispielsweise zunächst der nicht hydrolysierte "D-Ester" mit einem geeigneten organischen Lösungsmittel extrahiert und anschließend mit wäßriger Mineralsäure, wie Salzsäure verseift werden. Als Lösungsmittel bieten sich z. B. Hexan, Toluol, Xylol, oder Ether wie Diethylether oder Diisopropylether oder chlorierte Kohlenwasserstoffe wie Methylenchlorid, Chloroform oder Tetrachlorkohlenstoff an. Die in der wäßrigen Lösung verbleibende N-acylgeschützte "L -Säure" kann nach Ansäuern, vorzugsweise auf einen pH-Wert von 1-2, mit Lösungsmitteln wie Hexan, Toluol, Xylol, Essigester, Methylisobutylketon (MIBK) oder Ether wie Diethylether oder Diisopropylether oder chlorierten Kohlenwasserstoffen wie Methylenchlorid, Chloroform oder Tetrachlorkohlenstoff extrahiert werden. Auch die "L-Säure" wird anschließend mit wäßriger Mineralsäure verseift.

In den folgenden Beispielen wird die Erfindung näher erläutert.

**Beispiel 1**

8 g (27,7 mmol) D,L-N-Acetyl-3-trifluormethyl-phenylalaninmethylester werden in 40 ml MIBK gelöst und mit 160 ml Wasser versetzt. Die Mischung wird auf 45 °C gebracht und der pH-Wert auf 7 eingestellt. Dann wird 1 ml Maxatase (Gist-brocades) zugesetzt und kräftig gerührt. Um den pH-Wert konstant zu halten, wird kontinuierlich 0,5 N Natronlauge der Mischung zugeführt. Das Reaktionsende wird daran ersichtlich, daß die Natronlaugezugabe gegen Null geht. Die Reaktionszeit beträgt ca. 7 h.

Nach Beendigung der Reaktion werden die Phasen getrennt und zur vollständigen Isolierung des nicht umgesetzten "D-Esters" die wäßrige Phase noch dreimal mit 160 ml Essigester extrahiert. Anschließend wird die organische Phase über Natriumsulfat getrocknet und das Lösungsmittel abgezogen.

Ausbeute an D-N-Acetyl-3-trifluormethyl-phenylalaninmethylester: 3,98 g (99,5 % der Theorie).

Die wäßrige Phase wird auf pH = 1,5 gebracht und die "L-Säure" durch 4-maliges Extrahieren mit 160 ml Essigester isoliert. Anschließend wird die organische Phase über Natriumsulfat getrocknet und das Lösungsmittel abgezogen.

Ausbeute an L-N-Acetyl-3-trifluormethyl-phenylalanin: 3,7 g (98 % der Theorie).

**Beispiel 2**

28,9 g (100 mmol) des N-acetylgeschützten "D-Esters" aus Beispiel 1 werden in 440 ml 6 N Salzsäure 1 h unter Rückfluß gekocht. Danach läßt man die Lösung langsam auf Raumtemperatur abkühlen, wobei die "D-Säure" als Hydrochlorid auskristallisiert. Zur Vervollständigung der Kristallisation hält man die Lösung noch 2 h bei 4 °C, saugt ab und trocknet über Nacht bei 50 Torr und 80 °C.

Ausbeute an kristallinem D-3-Trifluormethyl-phenylalaninhydrochlorid: 22,9 g (85 % der Theorie, "ee" > 98 % lt. HPLC).

Das in der salzsauren Lösung verbliebene Material kann durch Abziehen des Wassers amorph erhalten werden und besitzt eine geringere optische Reinheit ("ee" ≈ 85 %).

**Beispiel 3**

Entsprechend Beispiel 2 werden auch 27,5 g (100 mmol) der aus Beispiel 1 stammenden N-acetylgeschützten "L-Säure" entschützt und als Hydrochlorid kristallisiert. Die Ausbeute an kristallinem L-3-Trifluormethyl-phenylalaninhydrochlorid beträgt 23,2 g (86 % der Theorie, "ee" > 98 % lt. HPLC). Das aus der salzsauren Lösung gewonnene Material hat auch hier eine geringere optische Reinheit ("ee" ≈ 85 %).

**Beispiel 4**

Entsprechend Beispiel 1 werden 8 g (31,1 mmol) D,L-N-Acetyl-2,4-difluorphenylalaninmethylester racematgespalten und aufgearbeitet. Die Reaktionszeit beträgt hier allerdings ca. 2 h. Die Ausbeute an D-N-Acetyl-2,4-difluorphenylalaninmethylester beträgt 4 g (100 % d. Th.), die an L-N-Acetyl-2,4-difluorphenylalanin beträgt 3,74 g (99 % der Theorie).

**Beispiel 5**

Entsprechend der Beispiele 2 und 3 werden jeweils 25,7 g bzw. 24,3 g (100 mmol) der 2,4-Difluor-Derivate entschützt.

Allerdings dürfen hier wegen der veränderten Kristallisationseigenschaften nur 330 ml 6 N Salzsäure für jeweils 100 mmol der Verbindungen verwendet werden. Die Ausbeuten an kristallinem D-2,4-Difluorphenylalanin und L-2,4-Difluorphenylalanin betragen jeweils 20,6 g (87 % der Theorie); der "ee"-Wert ist jeweils > 98 %. Auch hier liegen die optischen Ausbeuten der aus den salzsauren Lösungen nachträglich gewonnenen Materialien bei ca. 85 %.

**Beispiele 6 bis 12**

Analog zu den Beispielen 1-3 wurden die in der nachfolgenden Tabelle 1 aufgeführten Verbindungen erhalten (In den einzelnen Beispielen sind die g-Angaben entsprehend der Mol-Angaben auszurechenen. Alle übrigen Reaktionsparameter bleiben konstant.) Die chemischen Ausbeuten leigen immer zwischen 85 und 99 %; die optischen Ausbeuten oberhalb 98 %.

**Tabelle 1:**

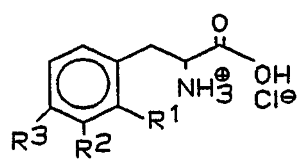

| | | | | Zers.-Punkt [°C] | |
|---|---|---|---|---|---|
| Bsp. | $R^1$ | $R^2$ | $R^3$ | D-Verbindung | L-Verbindung |
| 2,3 | H | $CF_3$ | H | 228-230 | 230-232 |
| 5 | F | H | F | 242-247 | 243-248 |
| 6 | H | H | $CF_3$ | 227-230 | 229-232 |
| 7 | F | H | H | 230-235 | 230-235 |
| 8 | H | F | H | 236-240 | 236-240 |
| 9 | H | H | F | 250-255 | 250-255 |
| 10 | H | H | n-Bu[1)] | 228-232 | 236-239 |
| 11 | H | H | Br | 247-249 | 250 |
| 12 | H | $CF_3$ | Cl | 244-250 | 243-250 |

[1)] n-Bu = n-Butyl

**Ansprüche**

1. Verfahren zur Herstellung optisch reiner phenylringsubstituierter Phenylalaninderivate durch racemische Spaltung mittels eines proteolytischen Enzyms, dadurch gekennzeichnet, daß eine racemische Verbindung der Formel I,

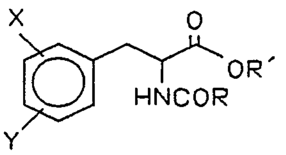

( I )

in der

X und Y gleich oder verschieden sind und Wasserstoff, Fluor, Chlor, Brom, ($C_1$-$C_4$)-Alkyl oder Trifluormethyl bedeuten und

R ($C_1$-$C_4$)-Alkyl und

R′ eine ($C_1$-$C_4$)-Alkyl- oder eine Benzylschutzgruppe bedeutet,

wobei X und Y niemals gleichzeitig Wasserstoff bedeuten und der Trifluormethyl-Substituent niemals die ortho-Position des Phenylrings besetzt, der Einwirkung von Subtilisin (EC 3.4.4.16) bei schwach saurem bis schwach alkalischem pH unterworfen wird.

2. Verfahren nach Anspruch 1, dadurch gekennzeichnet, daß die Umsetzung des N- und O-geschützten phenylringsubstituierten Phenylalaninderivats mit Subtilisin in Gegenwart eines Lösungsmittels, ausgewählt aus Wasser, Ethanol, Methanol oder den Mischungen Wasser/Methylisobutylketon, Wasser/Essigester, Wasser/Methanol und Wasser/Ethanol durchgeführt wird.

3. Verfahren nach Anspruch 1, dadurch gekennzeichnet, daß der pH-Wert 5,5 bis 8,5 beträgt.

4. Verfahren nach Anspruch 1, dadurch gekennzeichnet, daß die Umsetzung bei einer Reaktionstemperatur von 30-65° C durchgeführt wird.

5. Verfahren nach Anspruch 1, dadurch gekennzeichnet, daß Subtilisin zu racemischer Verbindung im Verhältnis 60 bis 600 U pro 100 g Substrat zugegeben wird.

6. Verfahren nach Anspruch 1, dadurch gekennzeichnet, daß eine racemische Verbindung der Formel I eingesetzt wird, in der

X Fluor in ortho , meta- oder para-Position oder Trifluormethyl in meta- oder para-Position oder Brom in para-Position

und

Y Wasserstoff bedeutet

oder

X Chlor in para-Position und Y Trifluormethyl in meta-Position bedeutet

oder

X n-Butyl in para-Position und Y Wasserstoff bedeutet

oder

X Fluor in para-Position und Y Fluor in ortho-Position bedeutet

und

R und R′ die im Anspruch 1 angegebene Bedeutung hat.

7. Verwendung der optisch reinen phenylringsubstituierten Phenylalaninderivate, erhältlich gemäß Verfahren nach Anspruch 1, zur Herstellung von Penicillinen, Cephalosporinen und Peptiden.